# EUROPEAN PATENT SPECIFICATION

(11) **EP 0 464 307 B1**
(45) Date of publication and mention of the grant of the patent: **10.01.1996**
(21) Application number: 91103159.9
(22) Date of filing: 28.03.1991
(51) Int. Cl.: C07C 273/04

(54) **Process and device to improve the performances and the life, with corrosion reduction, in Kettle type carbamate condensers in urea plants**
Verfahren und Einrichtung zur Verbesserung der Leistung und der Lebensdauer, mit Korrosionsverringerung, in Carbamatcondensoren der Kettle-Type in Harnstoffabriken
Procédé et dispositif pour améliorer les performances et la durée de vie, avec réduction de la corrosion, dans des condenseurs de carbamate du type Kettle dans des usines d'urée

(43) Date of publication of application: 08.01.1992
(73) Proprietor: UREA CASALE S.A., CH-6900 Lugano-Besso (CH)
(72) Inventor: Zardi, Umberto, CH-6932 Breganzona (CH)
(74) Representative: Checcacci, Giorgio

(56) References cited:
- EP-A- 0 002 298
- FR-A- 2 308 615
- GB-A- 2 157 687

## Description

The present invention relates to a process to improve the performances and to reduce the corrosion effects in high pressure and temperature condensation of the NH₃, CO₂, H₂O vapours, which are generated in the treatment systems, so-called of stripping, of the solution coming from the urea reactor, said condensation being carried out by means of a carbamate condenser in particular of the tube bundle "Kettle" type to which part of the carbamate solution effluents from the condenser itself is recycled.

As known in modern urea plants characterized by the isobaric stripping of most of the unreacted substances contained in the urea solution outflowing the reactor and which utilize as stripping agents part of the CO₂ or NH₃ feed stream, the so-obtained stripping vapours are condensed in suitable heat exchangers recovering the heat produced in said condensation with low pressure steam production (4ö5 bar a).

Regarding the condensation, it is necessary to feed the condenser with the carbamate solution coming from the downstream distillation section and containing enough water to keep the carbamate in solution too.

The carbamate condensers of particular interest are horizontal U-tube heat exchangers, better known as horizontal Kettle condensers usually utilized in the urea plants based on the Snamprogetti process.

This condensation stage is extremely critical and requires a complicated combination of operative parameters: it is in fact essential that the vapours to be condensed (NH₃, CO₂, H₂O) are finely mixed to the carbamate solution rich in water, which acts as absorbing agent, and comes from the section downstream the synthesis section, so that a high temperature condensation can take place producing therefore steam at the highest possible pressure; moreover it is necessary to protect the components (the condenser exchange surface), usually in stainless steel for costs reduction, from corrosion, being the process fluids very corrosive.

Unfortunately, the Kettle condensers, commonly used in modern plants too, denotes big life limitations and cannot assure optimal operative conditions, being based on processes which have various limitations and drawbacks, among them are cited:
- The separation (or segregation), at the condenser's pipe nozzle, of liquid-vapour components with consequent accumulation of vapours in the upper portion of the tube sheet of tube bundle and liquid in the lower portion of said sheet.
   This separation is caused by the low velocity of the fluids' circulation (approx. 0.1 m/s) and by the great difference in specific weight between liquid and vapour.
   Consequently, the vapour/liquid ratio in the zone facing the tube sheet and in most of the pipes shall not be optimal, and shall condensate with difficulty.
   To obviate this problem, it is necessary to notably increase the exchange surface, with incrementation of the size of the device and also of the cost for the same.
   In addition, the separation of the liquid-vapour components cause a lowering of the thermal level of the steam produced with respect to the amount theoretically producible.
- The corrosion of the tube sheet and the tubes (the exchange surface of the condenser in general) caused by the above mentioned separation of the two phases.
   One of the precautions normally undertaken to contain the negative effects of the phenomena is to introduce, for example, air into the system, which will reach the carbamate condenser through the stripping vapours to be condensed.
   It is, nevertheless, indispensable to guarantee a uniform passivation in all areas of the equipment avoiding zones with stagnant liquid or liquid circulating at a velocity insufficient to guarantee the necessary contribution of passivation oxygen (dead spots, creation of high temperature zones).
- An imperfect distribution of passivating air as described above that, being contained in the vapours, will tend to privilege the upper tubes of the carbamate condenser with respect to the lower ones.

Methods have been proposed, for example in the second integration of the Italian Patent Application No. 23214, in which is described a horizontal tube bundle condenser with recirculation outside the condenser of part of the carbamate solution effluent for the same apparatus this system being studied to solve the critical aspect of mixing of the absorption liquid and the vapors to be condensed in order to ensure an optimial distribution (steeping) of the liquid-steam mixture on the inside of the pipes of said condenser, that only partially and in an insatisfactory way solve the problems present in the above mentioned equipment.

In order to assure an optimal distribution of the liquid and vapour phases within the carbamate condenser, it has also been proposed by French application FR 2 308 615 to introduce the liquid/vapour mixture into a condensation zone including horizontal tubes, at a pressure of from 50 to 300 bar and under a hydrostatic head ΔP.

From here the importance of improving in a definitive way the performances and duration, with corrosion reduction in the carbamate condensers of Kettle type.

The first aim of the invention is to provide a process in which the above mentioned inconveniences are eliminated and a higher thermal level of produced steam is obtained, as well as a higher carbamate condensation, thus notably improving the performances and life of the equipment.

This aim and others still shall be better explained in the description that follows, are obtained through a process according to Claim 1 attached herewith.

According to a further aspect of the invention, the above-identified objects are achieved by a carbamate condenser for absorbing ammonia, carbon dioxide and water vapours by means of a water-rich carbamate solution as defined in the attached claim 8.

Another particular form of a device according to the invention is characterized by the fact that, when the Kettle-type carbamate condenser is vertical, the recycle of the carbamate inside the said zone facing the tube sheet is with thermosiphon circulation.

There are many advantages of operating in this manner, which are clearly shown in the following description.

The formation of carbamate in the operating conditions of the condenser are related more to the system's capacity to absorb the high reaction heat developed than to its kinetic nature; the higher this capacity is, the easier the formation of carbamate shall be.

In the zone preceding the tube inlet of the condenser, where the cold carbamate solution rich with water and hot vapours to be condensed come into contact, an almost instant reaction between the NH₃ and CO₂ takes place with formation of carbamate.

The adiabatic reaction is interrupted when the solution's temperature shall be in equilibrium with the vapour phase.

As a result there is a carbamate precondensation of a small portion of the CO₂, while the remaining portion shall be condensated in the condenser's tubes with the problems mentioned above.

In this particularly simple and advantageous way according to the process and the devices claimed, it is possible, being the recycle solution colder than the carbamate solution rich in water and therefore enabled to absorb heat, to notably increase the formation of carbamate in the zone facing the conderser tubes' inlet.

Among the many results obtained, we limit citing:
- a notable increase of liquid flow rate to the tubes;
- an elevated reduction of the vapours flow rate to the tubes;
- a consequent drastic reduction of the vapour/liquid ratio in the zone facing the tube inlet of the condenser.

Thanks to the higher liquid flow rate and the consequent higher pressure drop at the nozzle, it shall be possible to obtain a more uniform distribution of the fluids at the entrance of the tube sheet and inside the tubes, obtaining a higher thermal exchange coefficiency and a better vapours distribution, and therefore in the passivation air throughout the entire condenser, with practical elimination of the above mentioned corrosion phenomena. In addition, thanks to the higher thermal exchange coefficiency, it shall be possible to raise the thermal level of the steam produced.

The portion of pre-condensed carbamate in the zone facing the tube sheet, can be advantageously varied in order to obtain an almost complete condensation of the stripping vapours in the said zone preceding the pipes' inlet of the condenser.

Optimal results are, however, reachable limiting the above mentioned pre-condenser to a liquid/vapour weight ratio between 4 and 46, better between 4 and 20 and perferably between 5 and 10; in this way the ratio between recycle liquid and the carbamate solution rich in water is maintained in the interval between 4 and 30, better between 6 and 15 and better again between 6 and 10.

Is it particularly surprising how the inconveniences mentioned above can be brilliantly and simply resolved by modifying the Kettle type carbamate condenser already existing according to the present invention.

The different aspects and advantages of the invention are better shown in the detailed description of an example using references from the attached drawings, example given without any limitation to the invention, and in which :
- Figure 1 is a sketch of a section with partial axial schematic planes of a Kettle horizontal tube sheet condenser of traditional type;
- Figure 2 is a sketch of a section with partial axial schematic planes of the zone facing the tube sheet of the Kettle exchange of horizontal type with the indication of new devices according to a perferred version;
- Figure 3 is a sketch of a section with partial axial schematic planes of a Kettle condenser of vertical type having a carbamate recycle inside the zone facing the tube sheet with thermosiphon circulation.

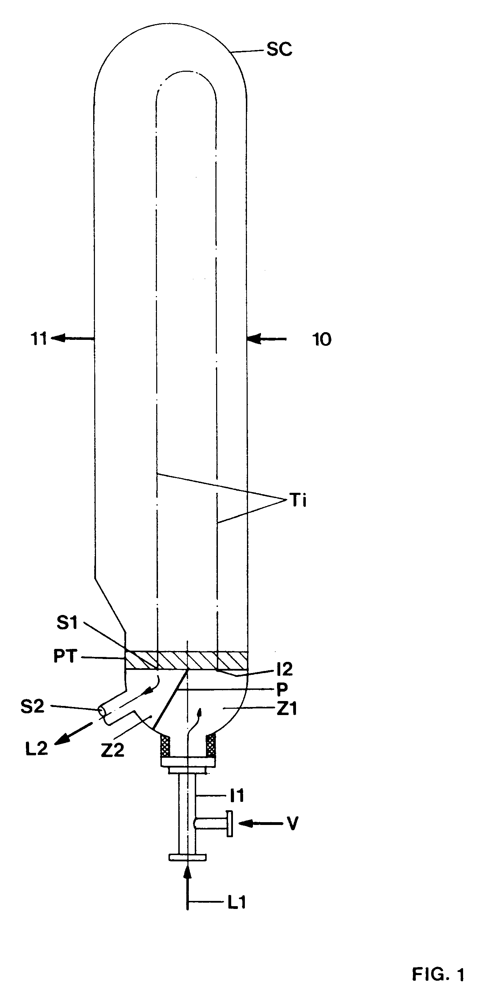

In particular, in Figure 1 a heat exchanger SC is shown, of horizontal Kettle tube sheet type with "U" pipes coming from a tube sheet PT at the inlet of the condenser SC, tube sheet that separates the tubes Ti from the influx zone Z1 and the discharge zone Z2 of the liquid-steam mixture, and then separate impermeabilized from the wall P.

The carbamate solution rich in water L1 coming from the distillation section downstream (not shown), and the stripping vapours V, flow together through the nozzle I1 in the chamber Z1, where they mix before they enter the tubes Ti, through the nozzle I2 of the tube sheet, from which they flow down through the outlet S1 of PT to then exit from the device, after having crossed Z2, through the opening S2.

The water for the thermal exchange is fed to the exchanger through line 10 and is collected in the form of acqueous vapour in line 11.

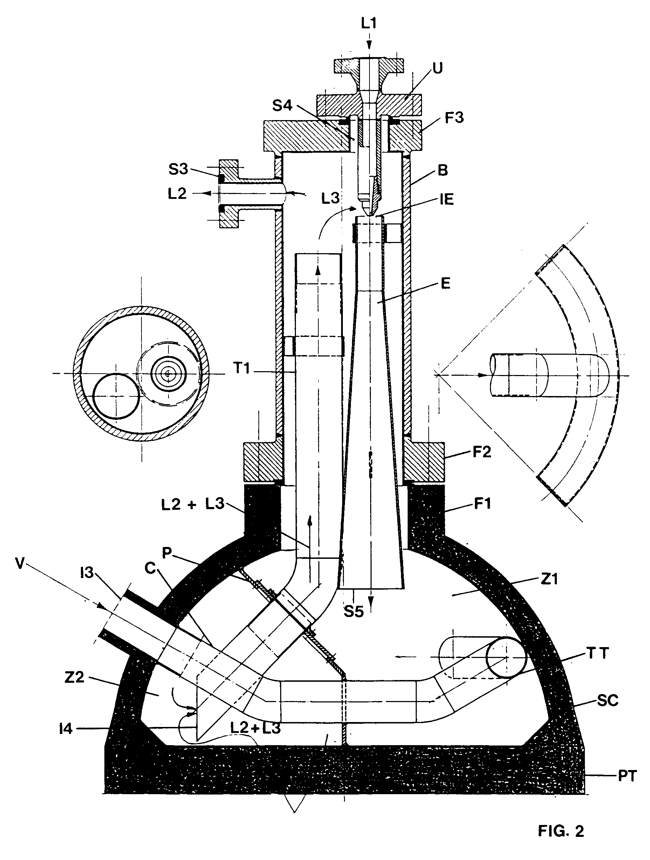

Figure 2 shows, in a detailed way, the portion facing said tube sheet PT of the exchanger SC, with the two zones Z1 and Z2 separate and impermeabilized from the wall P. To the flange F1 of said exchanger SC a feed opening B is connected with flanged ends F2, F3, sized to contain a return pipe T1 and an ejector E. The feed opening B is equipped with an outlet S3 for the condensed solution L2 to be sent to the reactor, and with an opening S4 for the insertion of an ejector nozzle U.

A conduct C is also foreseen to internally cross the zone Z1 and Z2, and with end terminating as torus section TT situated in the periphery of zone Z1 for an optimal dispersion of stripping vapours V which have entered from opening I3 of said conduct C.

The carbamate solution rich in water L1 (motor fluid) enters through the ejector nozzle U into B, is mixed at the opening IE of the ejector E with the recycle carbamate solution L3, and then enters from S5 into the zone Z1, where it comes into intimate contact with the stripping vapours V favouring the pre-condensation, before crossing the tube sheet PT.

The condensated mixture L2+L3 effluent PT enters in the opening I4 of the return pipe T1, put on the periphery of zone Z2 to avoid the accumulation of inerts (dead sacks), and is in part recycled (L3) on the inside of the condenser itself and in part (L2) sent to the synthesis reactor upstream (not shown).

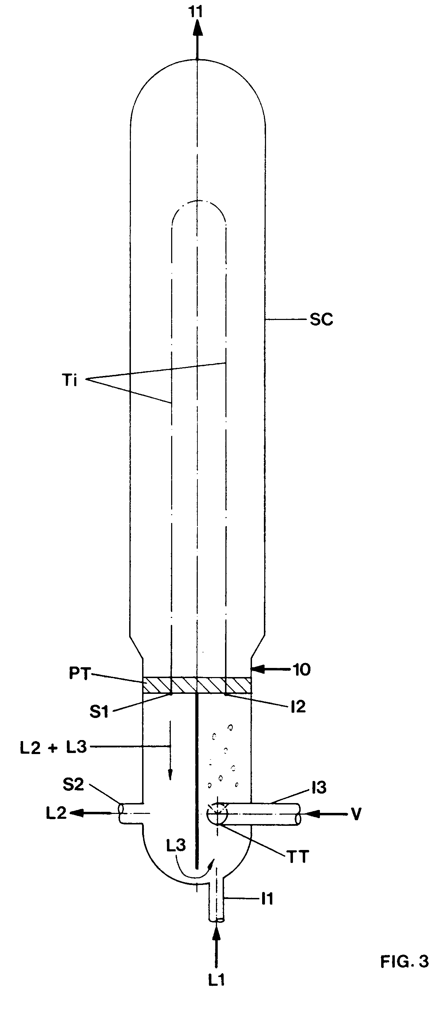

Figure 3 shows a Kettle-type carbamate condenser with vertical tube bundle evidencing the thermosiphon circulation of the carbamate recycle inside the zone facing the tube sheet.

The references used in Figure 1 and 2, and also shown in Figure 3, all obviously have the same meaning.

## Claims

1. A process for condensing ammonia, carbon dioxide and water vapours (V) generated by treating a solution coming from a urea reactor, said process comprising the steps of condensing the vapours (V) by means of a water-rich carbamate solution (L1) within a tube bundle carbamate condenser and recycling part (L3) of a condensed carbamate solution (L2 + L3) thus obtained to the condenser tubes (Ti),
characterized in that it further comprises the step of pre-condensing a large portion of said vapours (V) in front of the condenser tubes (Ti) inlet and in that the step of recycling part (L3) of the condensed carbamate solution (L2 + L3) is carried out within the condenser and upstream of said condenser tubes (Ti) inlet.

2. A process according to claim 1, characterized in that the liquid/vapour weight ratio at the condenser tubes (Ti) inlet varies from 4 to 46.

3. A process according to claim 2, characterized in that the liquid/vapour weight ratio at the condenser tubes (Ti) inlet varies from 5 to 10.

4. A process according to claim 1, characterized in that the weight ratio between the condensed carbamate solution (L3) recycled to the condenser tubes (Ti) and the water-rich carbamate solution (L1), is maintained in the range of from 4 to 30.

5. A process according to claim 4, characterized in that the weight ratio between the condensed carbamate solution (L3) recycled to the condenser tubes (Ti) and the water-rich carbamate solution (L1) is maintained in the range of from 6 to 10.

6. A process according to claim 1, characterized in that the partial recycle of the condensed carbamate solution (L2 + L3) is carried out within the condenser by means of an ejector (E).

7. A process according to claim 1, characterized in that the partial recycle of the condensed carbamate solution (L2 + L3) is carried out within the condenser by means of a termosiphon-type circulation.

8. A carbamate condenser for absorbing ammonia, carbon dioxide and water vapours (V) by means of a water-rich carbamate solution (L1) comprising:
- a heat exchanger (SC) including a bundle of U-shaped tubes (Ti) supported by means of a tube sheet (PT);
- a mixing chamber (Z1) in front of the tube sheet (PT) for mixing the water-rich carbamate solution (L1) with said ammonia, carbon dioxide and water vapours (V); and
- an outlet (S3) for discharging a condensed carbamate solution (L2) leaving said tube bundle;
characterized in that the condenser further comprises:
- an ejector (E) within said chamber (Z1), for mixing the water-rich carbamate solution (L1) with a portion (L3) of the condensed carbamate solution leaving said tube bundle (L2 + L3);
- a return pipe (T1) extending between the tube sheet (PT) and an inlet opening (IE) of said ejector (E), for recycling said portion (L3) of the condensed carbamate solution (L2 + L3); and
- a conduit (C) extending between a vapour inlet (I3) opening of the heat exchanger (SC) and the tube sheet (PT), for feeding said vapours (V) in front of the tube bundle inlet.

9. A condenser according to claim 8, characterized in that the return pipe (T1) comprises an inlet (I4) positioned proximate to a peripheral part (Z2) of the tube sheet (PT) and in front of the tubes (Ti) outlet.

10. A condenser according to claim 8, characterized in that said conduit (C) comprises a torus-shaped, perforated, end portion (TT), extending adjacent to a peripheral part (Z1) of the tube sheet (PT) and in front of the tubes (Ti) inlet.

11. A carbamate condenser for absorbing ammonia, carbon dioxide and water vapours (V) by means of a water-rich carbamate solution (L1) comprising:
- a heat exchanger (SC) including a vertical bundle of U-shaped tubes (Ti) supported by means of a tube sheet (PT);
- a mixing chamber (Z1) in front of the tube sheet (PT) for mixing the water-rich carbamate solution (L1) with said ammonia, carbon dioxide and water vapours (V); and
- an outlet (S3) for discharging a condensed carbamate solution (L2) leaving said vertical tube bundle;
characterized in that the condenser further comprises:
- a diaphragm which defines within said chamber (Z1) a liquid passageway proximate to an inlet nozzle (I1) of said water-rich carbamate solution (L1), said passageway providing means for recirculating and mixing a portion (L3) of the condensed carbamate solution (L2 + L3) with the water-rich carbamate solution (L1); and
- a conduit (C) extending between a vapour inlet (I3) opening of the heat exchanger (SC) and the tube sheet (PT), for feeding said vapours (V) upstream of the tubes (Ti) inlet.

## Patentansprüche

1. Verfahren zum Kondensieren von Ammoniak-, Kohlendioxid- und Wasserstoffdämpfen (V), die durch Behandeln einer von einem Harnstoffreaktor kommenden Lösung erzeugt worden sind, wobei das Verfahren die Schritte des Kondensierens der Dämpfe (V) mittels einer wasserreichen Karbamatlösung (L1) innerhalb eines Rohrbündel-Karbamatkondensators und des Zurückführens eines Teiles (L3) einer auf diese Weise gewonnenen kondensierten Karbamatlösung (L2 + L3) zu den Kondensatorenrohren (Ti) umfaßt, dadurch gekennzeichnet, daß es außerdem den Schritt des Vorkondensierens eines großen Anteils der Dämpfe (V) vor dem Einlaß der Kondensatorenrohre (Ti) umfaßt und daß der Schritt des Zurückführens des Teils (L3) der kondensierten Karbamatlösung (L2 + L3) innerhalb des Kondensators und stromaufwärts des Einlasses der Kondensatorenrohre (Ti) durchgeführt wird.

2. Verfahren nach Anspruch 1, dadurch gekennzeichnet, daß das Gewichtsverhältnis von Flüssigkeit zu Dampf an dem Einlaß der Kondensatorenrohre (Ti) von 4 bis 46 variiert.

3. Verfahren nach Anspruch 2, dadurch gekennzeichnet, daß das Gewichtsverhältnis von Flüssigkeit zu Dampf an dem Einlaß der Kondensatorenrohre (Ti) von 5 bis 10 variiert.

4. Verfahren nach Anspruch 1, dadurch gekennzeichnet, daß das Gewichtsverhältnis zwischen der zu den Kondensatorenrohren (Ti) zurückgeführten kondensierten Karbamatlösung (L3) und der wasserreichen Karbamatlösung (L1) in einem Wertebereich von 4 bis 30 gehalten wird.

5. Verfahren nach Anspruch 4, dadurch gekennzeichnet, daß das Gewichtsverhältnis zwischen der zu den Kondensatorenrohren (Ti) zurückgeführten kondensierten Karbamatlösung (L3) und der wasserreichen Karbamatlösung (L1) in einem Wertebereich von 6 bis 10 gehalten wird.

6. Verfahren nach Anspruch 1, dadurch gekennzeichnet, daß die Teilrückführung der kondensierten Karbamatlösung (L2 + L3) innerhalb des Kondensators mittels eines Ejektors (E) durchgeführt wird.

7. Verfahren nach Anspruch 1, dadurch gekennzeichnet, daß die Teilrückführung der kondensierten Karbamatlösung (L2 + L3) innerhalb des Kondensators mittels eines Umlaufs nach Art eines Thermosiphons durchgeführt wird.

8. Karbamatkodensator zur Aufnahme von Ammoniak-, Kohlendioxid- und Wasserdämpfen (V) mittels einer wasserreichen Karbamatlösung mit:
- einem Wärmetauscher (SC), der ein Bündel von U-förmigen Rohren (Ti) umfaßt, die mittels eines Rohrbodens (PT) abgestützt sind;
- einer Mischkammer (Z1) vor dem Rohrboden (PT) zum Mischen der wasserreichen Karbamatlösung (L1) mit den Ammoniak-, Kohlendioxid- und Wasserdämpfen (V); und
- einem Auslaß (S3) zum Ableiten einer aus dem Rohrbündel austretenden kondensierten Karbamatlösung (L2),
dadurch gekennzeichnet, daß der Kondensator außerdem aufweist:
- einen Ejektor (E) innerhalb der Kammer (Z1) zum Mischen der wasserreichen Karbamatlösung (L1) mit einem Teil (L3) der aus dem Rohrbündel austretenden kondensierten Karbamatlösung (L2 + L3);
- ein Rückflußrohr (T1), das sich zwischen dem Rohrboden (PT) und einer Einlaßöffnung (IE) des Ejektors (E) erstreckt, zum Zurückführen des Anteils (L3) der kondensierten Karbamatlösung (L2 + L3); und
- ein Rohr (C) zum Zuführen der Dämpfe (V) vor den Rohrbündeleinlaß, das sich zwischen einer Dampfeinlaßöffnung (I3) des Wärmetauschers (SC) und dem Rohrboden (PT) erstreckt.

9. Kondensator nach Anspruch 8, dadurch gekennzeichnet, daß das Rückflußrohr (T1) einen Einlaß (I4) umfaßt, der nahe eines Umfangsabschnittes (Z2) des Rohrbodens (PT) und vor dem Auslaß der Rohre (Ti) angeordnet ist.

10. Kondensator nach Anspruch 8, dadurch gekennzeichnet, daß das Rohr (C) einen torusförmigen, perforierten Endbereich (TT) umfaßt, der sich angrenzend an einem Umfangsabschnitt (Z1) des Rohrbodens (PT) und vor dem Einlaß der Rohre (Ti) erstreckt.

11. Karbamatkodensator zur Aufnahme von Ammoniak-, Kohlendioxid- und Wasserdämpfen (V) mittels einer wasserreichen Karbamatlösung mit:
- einem Wärmetauscher (SC), der ein vertikales Bündel von U-förmigen Rohren (Ti) umfaßt, die mittels eines Rohrbodens (PT) abgestützt sind;
- einer Mischkammer (Z1) vor dem Rohrboden (PT) zum Mischen der wasserreichen Karbamatlösung (L1) mit den Ammoniak-, Kohlendioxid- und Wasserdämpfen (V); und
- einem Auslaß (S3) zum Ableiten einer aus dem Rohrbündel austretenden kondensierten Karbamatlösung (L2),
dadurch gekennzeichnet, daß der Kondensator außerdem aufweist:
- eine Membrane, die innerhalb der Kammer (Z1) einen Flüssigkeitsdurchgang in der Nähe einer Einlaßdüse (I1) der wasserreichen Karbamatlösung (L1) bildet, wobei der Durchgang Mittel zum Rezirkulieren und Mischen eines Anteils (L3) der kondensierten Karbamatlösung (L2 + L3) mit der wasserreichen Karbamatlösung (L1) bereitstellt; und
- ein Rohr (C) zum Zuführen der Dämpfe (V) stromaufwärts von dem Einlaß der Rohre (Ti), das sich zwischen einer Dampfeinlaßöffnung (I3) des Wärmetauschers (SC) und dem Rohrboden (PT) erstreckt.

## Revendications

1. Procédé pour condenser des vapeurs de gaz ammoniac, de dioxyde de carbone et d'eau (V) produites en traitant une solution provenant d'un réacteur d'urée, ledit procédé comprenant les étapes de condensation des vapeurs (V) au moyen d'une solution de carbamate riche en eau (L1) à l'intérieur d'un condenseur de carbamate à faisceau tubulaire, et de recyclage d'une partie (L3) d'une solution de carbamate condensée (L2 + L3) ainsi obtenue vers les tubes de condenseur (Ti),
caractérisé en ce qu'il comprend, de plus, l'étape de précondensation d'une grande partie desdites vapeurs (V) devant l'entrée des tubes de condenseur (Ti) et en ce que l'étape de recyclage de la partie (L3) de la solution de carbamate condensée (L2 + L3) est exécutée à l'intérieur du condenseur et en amont de ladite entrée des tubes de condenseur (Ti).

2. Procédé selon la revendication 1, caractérisé en ce que le rapport de poids liquide/vapeur, au niveau de l'entrée des tubes de condenseur (Ti) varie de 4 à 46.

3. Procédé selon la revendication 2, caractérisé en ce que le rapport de poids liquide/vapeur, au niveau de l'entrée des tubes de condenseur (Ti) varie de 5 à 10.

4. Procédé selon la revendication 1, caractérisé en ce que le rapport de poids, entre la solution de carbamate condensée (L3) recyclée vers les tubes de condenseur (Ti) et la solution de carbamate riche en eau (L1), est maintenu dans la plage de 4 à 30.

5. Procédé selon la revendication 4, caractérisé en ce que le rapport de poids, entre la solution de carbamate condensée (L3) recyclée vers les tubes de condenseur (Ti) et la solution de carbamate riche en eau (L1), est maintenu dans la plage de 6 à 10.

6. Procédé selon la revendication 1, caractérisé en ce que le recyclage partiel de la solution de carbamate condensée (L2 + L3) est exécuté à l'intérieur du condenseur au moyen d'un éjecteur (E).

7. Procédé selon la revendication 1, caractérisé en ce que le recyclage partiel de la solution de carbamate condensée (L2 + L3) est exécuté à l'intérieur du condenseur au moyen d'une circulation du type à thermosiphon.

8. Condenseur de carbamate pour absorber les vapeurs de gaz ammoniac, de dioxyde de carbone et d'eau (V) au moyen d'une solution de carbamate riche en eau (L1) comprenant :
- un échangeur de chaleur (SC) incluant un faisceau de tubes en forme de U (Ti) supportés au moyen d'une plaque tubulaire (PT) ;
- une chambre de mélange (Z1) en face de la plaque tubulaire (PT) pour mélanger la solution de carbamate riche en eau (L1) avec lesdites vapeurs de gaz ammoniac, de dioxyde de carbone et d'eau (V) ; et
- un orifice de sortie (S3) pour évacuer une solution de carbamate condensée (L2) quittant ledit faisceau tubulaire ;
caractérisé en ce que le condenseur comprend, de plus :
- un éjecteur (E) à l'intérieur de ladite chambre (Z1), pour mélanger la solution de carbamate riche en eau (L1) avec une partie (L3) de la solution de carbamate condensée quittant ledit faisceau tubulaire (L2 + L3) ;
- un tuyau de retour (T1) s'étendant entre la plaque tubulaire (PT) et une ouverture d'entrée (IE) dudit éjecteur (E), pour recycler ladite partie (L3) de la solution de carbamate condensée (L2 + L3) ; et
- un conduit (C) s'étendant entre une ouverture d'entrée de vapeur (I3) de l'échangeur de chaleur (SC) et la plaque tubulaire (PT), pour délivrer lesdites vapeurs (V) en face de l'entrée du faisceau tubulaire.

9. Condenseur selon la revendication 8, caractérisé en ce que le tuyau de retour (T1) comprend une entrée (I4) positionnée à proximité d'une partie périphérique (Z2) de la plaque tubulaire (PT) et en face de la sortie des tubes (Ti).

10. Condenseur selon la revendication 8, caractérisé en ce que ledit conduit (C) comprend une partie d'extrémité (TT), perforée, en forme de tore, s'étendant à côté d'une partie périphérique (Z1) de la plaque tubulaire (PT) et en face de l'entrée des tubes (Ti).

11. Condenseur de carbamate pour absorber les vapeurs de gaz ammoniac, de dioxyde de carbone et d'eau (V) au moyen d'une solution de carbamate riche en eau (L1) comprenant :
- un échangeur de chaleur (SC) incluant un faisceau vertical de tubes en forme de U (Ti) supportés au moyen d'une plaque tubulaire (PT) ;
- une chambre de mélange (Z1) en face de la plaque tubulaire (PT) pour mélanger la solution de carbamate riche en eau (L1) avec lesdites vapeurs de gaz ammoniac, de dioxyde de carbone et d'eau (V) ; et
- un orifice de sortie (S3) pour évacuer une solution de carbamate condensée (L2) quittant ledit faisceau tubulaire vertical ;
caractérisé en ce que le condenseur comprend, de plus :
- un diaphragme qui définit à l'intérieur de ladite chambre (Z1) une voie de passage de liquide proche d'une buse d'entrée (I1) de ladite solution de carbamate riche en eau (L1), ladite voie de passage offrant un moyen pour faire recirculer et mélanger une partie (L3) de la solution de carbamate condensée (L2 + L3) avec la solution de carbamate riche en eau (L1) ; et
- un conduit (C) s'étendant entre une ouverture d'entrée de vapeur (I3) de l'échangeur de chaleur (SC) et la plaque tubulaire (PT), pour délivrer lesdites vapeurs (V) en amont de l'entrée des tubes (Ti).
